(19) Europäisches Patentamt / European Patent Office / Office européen des brevets

(11) **EP 2 889 617 B1**

(12) **EUROPEAN PATENT SPECIFICATION**

(45) Date of publication and mention
of the grant of the patent:
**11.10.2017 Bulletin 2017/41**

(21) Application number: **13832456.1**

(22) Date of filing: **27.08.2013**

(51) Int Cl.:
**G01N 30/88** *(2006.01)*     **C07K 1/22** *(2006.01)*
**G01N 30/26** *(2006.01)*     **G01N 30/54** *(2006.01)*
**B01D 15/38** *(2006.01)*

(86) International application number:
**PCT/JP2013/072821**

(87) International publication number:
**WO 2014/034644 (06.03.2014 Gazette 2014/10)**

(54) **ANTIBODY PURIFICATION METHOD BY MEANS OF TEMPERATURE-RESPONSIVE CHROMATOGRAPHY**

ANTIKÖRPERREINIGUNGSVERFAHREN MITTELS TEMPERATUREMPFINDLICHER CHROMATOGRAPHIE

PROCÉDÉ DE PURIFICATION D'ANTICORPS AU MOYEN D'UNE CHROMATOGRAPHIE SENSIBLE À LA TEMPÉRATURE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.08.2012 JP 2012186930**
**16.04.2013 JP 2013086121**

(43) Date of publication of application:
**01.07.2015 Bulletin 2015/27**

(73) Proprietor: **Asahi Kasei Medical Co., Ltd.**
**Chiyoda-ku**
**Tokyo 101-8101 (JP)**

(72) Inventors:
• **KOGUMA, Ichiro**
**Tokyo 101-8101 (JP)**
• **ISHIKAWA, Rumiko**
**Tokyo 101-8101 (JP)**
• **TAKEMASA, Hiroshi**
**Tokyo 101-8101 (JP)**
• **OKUYAMA, Kazuo**
**Tokyo 101-8101 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**EP-A1- 0 984 022     EP-A1- 1 281 436**
**WO-A1-2008/143199     WO-A1-2011/017514**
**WO-A1-2012/086837     WO-A1-2012/086838**
**WO-A1-2012/086838     JP-A- 2007 526 897**
**JP-A- 2009 196 998     US-A1- 2010 168 395**

• **SCHULER G ET AL: "Development and optimization of a single-step procedure using protein A affinity chromatography to isolate murine IgG1 monoclonal antibodies from hybridoma supernatants", JOURNAL OF CHROMATOGRAPHY, ELSEVIER SCIENCE PUBLISHERS B.V, NL, vol. 587, no. 1, 29 November 1991 (1991-11-29), pages 61-70, XP026539184, ISSN: 0021-9673, DOI: 10.1016/0021-9673(91)85198-O [retrieved on 1991-11-29]**

**Description**

Technical Field

The present invention relates to a method for purifying an antibody by utilizing temperature-responsive chromatography which exhibits a change in binding property to the antibody along with changes in temperature.

Background Art

Immunoglobulins (antibodies) are physiologically active substances taking part in the immune reactions. Utility values of antibodies have recently been elevated in applications to separation and purification materials and the like for pharmaceutical preparations, diagnostic reagents and corresponding antigenic proteins.
Antibodies are acquired from blood of immunized animals, cell culture media of cells retaining the antibody production ability, or ascites culture media of animals. Blood and culture media containing these antibodies, however, contain proteins other than the antibodies or complex contaminants originated from raw material liquids used for cell culture, and in order to separate and purify antibodies from these impurity components, complex operations taking a long time are usually needed.
Liquid chromatography is important for the separation and purification of antibodies. Chromatographic techniques to separate antibodies includes gel filtration chromatography, affinity chromatography, ion exchange chromatography and reversed phase chromatography, and antibodies are separated and purified by combining these techniques.
In affinity chromatography, an antibody having a high purity and a high concentration is acquired by purification through steps of the following (A) to (C).

(A) A step (loading step) of loading a sample of a mixture of the antibody and impurities on a column.
(B) A step (washing step) of removing the impurities excluding the antibody as a purification object from the loaded column.
(C) A step (elution step) of eluting and recovering the antibody as the purification object from the column.

In recent years, as a ligand of affinity chromatography to purify antibodies, Protein A has attracted attention. Protein A is derived from Staphylococcus aureus. Protein A has a high affinity specific for the Fc region of antibodies under a neutral condition. Hence, when an antibody is purified using natural Protein A, a solution containing the antibody is brought into contact with a stationary phase having the natural Protein A as a ligand under a neutral condition to thereby make the antibody to be specifically adsorbed on the natural Protein A on a medium. Then, components not having being adsorbed on the medium are washed and removed with a neutral buffer solution, and thereafter, the antibody is released from the natural Protein A on the medium using a solution of an acidity of a pH of nearly 3.0 (see, for example, Patent Literatures 1, 2). In Patent Literature 1, an antibody is eluted from a Protein A medium using an acidic solution composed of a 25 mM sodium citrate (pH: 2.8), in Examples. Also in Patent Literature 2, in Examples, an antibody is eluted from a Protein A medium using an acidic solution composed of a 25 mM citrate salt (pH: 2.8) or a 0.1 M acetic acid (pH: 2.9). Under such a strong acidic condition, there arises a risk that denaturation and inactivation could be caused to antibodies as a purification object.
In order to change the affinity of natural Protein A for antibodies in response to the change in the hydrogen ion exponent of a buffer solution as described above, antibodies as a purification object need to be exposed to an acidic condition. Therefore, the activity of the antibodies as a purification object is impaired in some cases. By contrast, methods for purifying antibodies are proposed which use variant Protein A (temperature-responsive Protein A) whose affinity for antibodies changes due to the conformational change and the like along with the temperature change without any change in the hydrogen ion exponent of a buffer solution (see, for example, Patent Literatures 3, 4). Since use of temperature-responsive Protein A as a ligand of affinity chromatography makes it unnecessary for a buffer solution to be made acidic, the activity of antibodies can be retained. In purification of antibodies using a temperature-responsive Protein A medium, however, studies on means to enhance the removability of impurities such as host cell-originated proteins (HCP) and deoxyribonucleic acids (DNA), and to suppress the detachment of Protein A have not advanced.

Citation List

Patent Literature

Patent Literature 1: Japanese Patent Laid-Open No. 2009-196998

Patent Literature 2: National Publication of International Patent Application No. 2007-526897
Patent Literature 3: International Publication No. WO 2008/143199
Patent Literature 4: International Publication No. WO 2011/017514

Summary of Invention

Technical Problem

In methods for purifying antibodies using temperature-responsive Protein A, the removal of impurities possibly remaining in affinity columns, such as host cell-derived proteins (HCP) and deoxyribonucleic acids (DNA), is demanded. The suppression of mingling of the temperature-responsive Protein A detached from the medium into an eluent of the antibodies is also demanded.

Also in the methods for purifying antibodies using temperature-responsive Protein A, the temperature needs to be raised to about 40°C when the antibodies are eluted, but setting the temperature high has brought about a reduction in the activity of the antibodies to some extent so far unavoidably. Although, in order to avoid the reduction in the activity of the antibodies, making the temperature range where the antibodies are released from temperature-responsive Protein A to be changed to a lower temperature side is effective, no studies on means to realize this have been made so far. WO2012/086837 discloses a method for the purification of antibodies using temperature-responsive protein A.

Then, the present invention has an object to provide a method for purifying an antibody using temperature-responsive Protein A attached to a medium, in which method impurities are effectively removed and the detachment of the temperature-responsive Protein A from the medium is little. The present invention further has an object to provide a method for purifying an antibody using temperature-responsive Protein A, which method can maintain the activity of the antibody high.

Solution to Problem

In the case where a stationary phase has natural Protein A, different buffer solutions conventionally need to be used in a washing step and an elution step. By contrast, in the case where a stationary phase has temperature-responsive Protein A, it is conventionally considered as an advantage that the same buffer solution can be used in a washing step and an elution step. Therefore, in the case where a stationary phase has temperature-responsive Protein A, use of buffer solutions different in at least one of the salt concentration and the hydrogen ion exponent in a washing step and an elution step has not been studied.

Further in purification of an antibody using temperature-responsive Protein A, the influence of the salt concentration and the hydrogen ion exponent of a buffer solution used in an elution step on the elution temperature also has not been studied.

As a result of exhaustive studies, however, the present inventors have found that in a method for purifying an antibody using temperature-responsive Protein A attached to a medium, the use of a buffer solution having a high salt concentration in a washing step and a buffer solution having a low salt concentration in an elution step enables impurities to be effectively removed and the detachment of the temperature-responsive Protein A from the medium to be suppressed. The present inventors have further found that also the use of a buffer solution having a high hydrogen ion exponent in a washing step and a buffer solution having a low hydrogen ion exponent in an elution step enables impurities to be effectively removed and the detachment of the temperature-responsive Protein A from the medium to be suppressed.

The present inventors have further found such a phenomenon that the use of a buffer solution having a low hydrogen ion exponent in an elution step changes a temperature range where an antibody is released from temperature-responsive Protein A to a lower temperature side. It has been found from this that in an elution step of the purification of an antibody using temperature-responsive Protein A, the use of a buffer solution having a low hydrogen ion exponent enables the antibody to be eluted at a lower temperature than conventionally.

According to an aspect according to the present invention based on at least one of the above-mentioned first findings by the present inventors, a method for purifying an antibody using temperature-responsive Protein A is provided, which comprises a binding step of binding the antibody to a stationary phase having the temperature-responsive Protein A, a washing step of washing the stationary phase using a buffer solution having a first salt concentration at a temperature at which the temperature-responsive Protein A and the antibody are bound, and an elution step of eluting the antibody captured by the stationary phase using a buffer solution having a second salt concentration lower than the first salt concentration at a temperature at which the antibody is released from the temperature-responsive Protein A, wherein the different buffer solutions are used in the washing step and the elution step.

A method for purifying an antibody using temperature-responsive Protein A is further provided which comprises a binding step of binding the antibody to a stationary phase having the temperature-responsive Protein A, a washing step of washing the stationary phase using a buffer solution having a first hydrogen ion exponent at a temperature at which the temperature-responsive Protein A and the antibody are bound, and an elution step of eluting the antibody captured by the stationary phase using a buffer solution having a second hydrogen ion exponent lower than the first hydrogen ion exponent at a temperature at which the antibody is released from the temperature-responsive Protein A, wherein the different buffer solutions are used in the washing step and the elution step.

Advantageous Effects of Invention

The present invention provides a method for purifying an antibody using temperature-responsive Protein A attached to a medium, in which method for purifying the antibody impurities are effectively removed and the detachment of the temperature-responsive Protein A from the medium is little. The present invention can further provide a method for purifying an antibody using temperature-responsive Protein A, which method can maintain the activity of the antibody high.

Description of Embodiment

Hereinafter, an embodiment (hereinafter, referred to as "the present embodiment") according to the present invention will be described in detail. Here, the embodiment described below only exemplifies an apparatus and a method for embodying the technical idea according to the present invention, and the technical idea according to the present invention is not specified to the following. With respect to the technical idea according to the present invention, various changes and modifications may be made within the scope of the claims.

A method for purifying an antibody using temperature-responsive Protein A according to the present embodiment comprises a binding step of binding the antibody to a stationary phase having the temperature-responsive Protein A, a washing step of washing the stationary phase using a buffer solution having a first salt concentration at a temperature at which the temperature-responsive Protein A and the antibody are bound, and an elution step of eluting the antibody captured by the stationary phase using a buffer solution having a second salt concentration lower than the first salt concentration at a temperature at which the antibody is released from the temperature-responsive Protein A, wherein the different buffer solutions are used in the washing step and the elution step.

Further a method for purifying an antibody using temperature-responsive Protein A according to the present embodiment comprises a binding step of binding the antibody to a stationary phase having the temperature-responsive Protein A, a washing step of washing the stationary phase using a buffer solution having a first hydrogen ion exponent at a temperature at which the temperature-responsive Protein A and the antibody are bound, and an elution step of eluting the antibody captured by the stationary phase using a buffer solution having a second hydrogen ion exponent lower than the first hydrogen ion exponent at a temperature at which the antibody is released from the temperature-responsive Protein A, wherein different buffer solutions are used in the washing step and the elution step.

The stationary phase having temperature-responsive Protein A has the temperature-responsive Protein A and a medium to which the temperature-responsive Protein A is attached. The temperature-responsive Protein A has a property of binding an antibody in a low-temperature range and releasing the antibody in a high-temperature region. The shape of the medium is not especially limited, and includes, for example, a film shape such as a flat film shape and a hollow fiber shape, and a bead shape. A hollow fiber-shape medium, since being easily shaped into a module and having a large film area capable of being packed per module container, can suitably be used. Further a bead-shape one, in general, since having a larger surface area per volume than a film-shape one and being capable of adsorbing a large amount of antibodies, can suitably be used.

The material of the medium is not especially limited, and in the case where the medium is the film-shape one, a polymer material capable of being formed into a porous film can suitably be used. Examples thereof usable are olefin resins such as polyethylene and polypropylene, polyester resins such as polyethylene terephthalate and polyethylene naphthalate, polyamide resins such as nylon 6 and nylon 66, fluorine-containing resins such as polyvinylidene fluoride and polychlorotrifluoroethylene, and noncrystalline resins such as polystyrene, polysulfone, polyether sulfone and polycarbonate. In the case where the medium is of the bead shape, as a material of the medium, usable are glass, silica, polystyrene resins, methacrylic resins, crosslinked agarose, crosslinked dextran, crosslinked polyvinyl alcohols and crosslinked cellulose. The crosslinked polyvinyl alcohols and the crosslinked cellulose, since having a high hydrophilicity and being capable of suppressing adsorption of impurity components, can suitably be used.

The medium used in the present embodiment has, for example, a plurality of pores. The pore diameter is not especially limited, but is, for example, 5 to 1,000 nm, preferably 10 to 700 nm, and more preferably 20 to 500 nm. When the pore diameter is 5 nm or smaller, the molecular weight of separable antibodies is likely to become low. When the pore diameter is 1,000 nm or larger, the surface area of a base material becomes small and the binding capacity of antibodies is likely to become low.

An optional coupling group may be introduced to the medium. The coupling group, since temperature-responsive Protein A has primary amino groups, is preferably an NHS-activated carboxyl group, a carboxyl group, a cyanogen bromide-activated group, an epoxy group, a formyl group or the like, which can be coupled with a primary amino group. Particularly the NHS-activated carboxyl group needs no other chemical in the coupling reaction and exhibits a rapid reaction, and is suitably used to form a firm bond.

In the present embodiment, a method for introducing the coupling group to the medium may be any method, but it is usual that a spacer is introduced between the medium and the coupling group. Methods for introducing a coupling group are disclosed in various literatures.

In the present embodiment, a graft polymer chain having coupling groups on terminals and/or side chains thereof may be introduced to the medium. The introduction of the graft polymer chain having coupling groups to the medium enables the density of the coupling group to be controlled, for example, to be optionally raised. A polymer chain having coupling groups may be grafted to the medium, or a polymer chain having precursor functional groups being capable of being converted to coupling groups may be grafted to the medium and the grafted precursor functional groups may then be converted to the coupling groups.

A method for introducing the graft polymer chain may be any method. The polymer chain may be prepared in advance and coupled to the medium. Alternatively, the graft chain may be polymerized directly on the medium by means of a "living radical polymerization method" or a "radiation-induced graft polymerization method". The "radiation-induced graft method", since no reaction initiator needs to be introduced in advance to the medium, and many kinds of mediums are applicable, can suitably be used.

In the case where the graft chain is introduced by the "radiation-induced graft polymerization method", any means to produce radicals on the medium can be employed, but in order to uniformly produce radicals on the entire medium, irradiation with an ionizing radiation is preferable. Kinds utilizable of ionizing radiation are γ rays, electron beams, β rays, neutron beams and the like, but for practicing in industrial scales, electron beams and γ rays are preferable. The ionizing radiation can be obtained from a radioisotope such as cobalt-60, strontium-90 or cesium-137, or an X-ray radiographic apparatus, an electron accelerator, an ultraviolet irradiation apparatus or the like.

The exposure dose of the ionizing radiation is, for example, preferably 1 kGy or more and 1,000 kGy or less, more preferably 2 kGy or more and 500 kGy or less, and still more preferably 5 kGy or more and 200 kGy or less. With the exposure dose being less than 1 kGy, radicals are likely to be hardly uniformly produced. With the exposure dose exceeding 1,000 kGy, the physical strength of a medium is likely to be caused to decrease.

The graft polymerization method using irradiation with the ionizing radiation is usually broadly divided into a previous irradiation method in which radicals are produced on the medium, and thereafter, the radicals are brought into contact with a reactive compound, and a simultaneous irradiation method in which radicals are produced on the medium in the state that the medium is being brought into contact with a reactive compound. In the present embodiment, any method may be applicable, but the previous irradiation method, which provides little oligomer, is preferable.

A solvent used in the graft polymerization in the present embodiment is not especially limited as long as being capable of dissolving homogeneously the reactive compound. Examples of such solvents include alcohols such as ethanol, isopropanol and t-butyl alcohol; ethers such as diethyl ether and tetrahydrofuran; ketones such as acetone and 2-butanone; water; and mixtures thereof.

A monomer having the coupling group used in the graft polymerization in the present embodiment, in the case of using a carboxyl group as the coupling group, includes monomers such as acrylic acid and methacrylic acid, and in the case of using a primary amino group as the coupling group, includes allylamine, and in the case of using an epoxy group as the coupling group, includes glycidyl methacrylate.

In the present embodiment, a monomer having the precursor functional group capable of being converted to the coupling group may be grafted to the medium, and thereafter, the grafted precursor functional group may be converted to the coupling group. Glycidyl methacrylate (GMA), which has an epoxy group, since being capable of being converted to various functional groups by utilizing various ring-opening reactions of the epoxy group, can be used suitably also industrially.

In the case of using the carboxyl group as the coupling group, GMA is first graft polymerized; thereafter, the epoxy group of GMA is hydrolyzed to thereby make a diol; and a cyclic acid anhydride is subjected to a ring-opening half-esterification reaction with the hydroxyl group originated from the diol to be thereby able to form (ring-opening half-esterification reaction) the carboxyl group originated from the cyclic acid anhydride. The cyclic acid anhydride is desirably succinic anhydride or glutaric anhydride in the point of the production cost, but is not limited thereto.

A catalyst to be used in the ring-opening half-esterification reaction is not especially limited as long as promoting the reaction, but specifically includes triethylamine, isobutylethylamine, pyridine and 4-dimethylaminopyridine; triethylamine or 4-dimethylaminopyridine is preferable; and 4-dimethylaminopyridine is most preferable in the point of the reaction speed and yield.

The ring-opening half-esterification reaction is carried out preferably in an inactive organic solvent, such as toluene, having the catalyst added thereto.

The NHS-activation reaction in the present embodiment is a step of converting the carboxyl group formed in the ring-opening half-esterification reaction to an active ester. Since the reactivity of the active ester is higher than that of the carboxyl group, in the case where it is desired that temperature-responsive Protein A is rapidly fixed on the medium, the active-esterification step is preferably carried out.

The active ester performs a function of bonding a hydrophilic compound to a substance to be fixed on through a covalent bond. Here, the active ester means a chemical structure of R-C(=O)-X. X is a leaving group such as a halogen, an N-hydroxysuccinimide group or its derivative, a 1-hydroxybenzotriazole group or its derivative, a pentafluorophenyl group or a paranitrophenyl group, but is not limited thereto. As the active ester, an N-hydroxysuccinimide ester is desirable in the point of the reactivity, the safety and the production cost. The conversion of the carboxyl group to an N-hydroxysuccinimide ester can be achieved by reacting the carboxyl group simultaneously with N-hydroxysuccinimide and a carbodiimide. Here, the carbodiimide means an organic compound having a chemical structure of -N=C=N-, and includes, for example, dicyclohexylcarbodiimide, diisopropylcarbodiimide and a 1-ethyl-3-(3-dimethylaminopropyl)carbodiimide hydrochloride, but is not limited thereto. It is desirably set that the concentrations of N-hydroxysuccinimide and the carbodiimide are in the range of 1 to 100 mmol/L; the reaction temperature is in the range of 0°C or higher and lower than 100°C; the reaction time is in the range of 2 min to 16 hours. As a reaction solvent, N,N'-dimethylformamide (DMF), toluene or the like can be used.

In the present embodiment, Protein A varied so as to change the binding property to an antibody depending on the temperature can be regulated by reference to a Patent Literature (WO2008/143199).

In the present embodiment, the coupling reaction of the NHS-activated carboxyl group with the temperature-responsive Protein A is carried out, for example, as follows. A temperature-responsive Protein A solution of 0.1 to 100 mg/mL is first prepared using a buffer solution containing no amino group component, such as a citrate buffer solution (pH: 3.0 to 6.2), an acetic acid buffer solution (pH: 3.6 to 5.6), a phosphoric acid-buffered saline (PBS, pH: 5.8 to 8.5) or a carbonic acid buffer solution (pH: 9.2 to 10.6). When the solution is brought into contact with a surface of active esters, functional groups such as amino groups contained in the temperature-responsive Protein A are reacted with the active esters to thereby form amide bonds. As a result, the temperature-responsive Protein A is fixed to the medium through a covalent bond. Here, the contact time may be set in the range of 2 min to 16 hours. After the temperature-responsive Protein A is fixed to the medium, the medium is desirably washed with a proper washing solution. At this time, if a buffer solution containing a salt (NaCl) of about 0.5 mol/L and about 0.1% of a nonionic surfactant is used as the washing solution, the temperature-responsive Protein A not being covalently bonded to the medium and only being physically adsorbed thereon can be removed, which is preferable.

After the temperature-responsive Protein A is fixed to the medium surface (preferably further after the temperature-responsive Protein A-fixed medium is washed), it is preferable that unreacted carboxyl groups or active esters be converted to functional groups having a lower reactivity by binding a low-molecular weight compound having an amino group to the unreacted carboxyl groups or active esters. Molecules other than the purification object, such as impurities, can thereby be prevented from being unintendedly bound to the stationary phase. Particularly in the case where terminal functional groups of the medium for fixing the temperature-responsive Protein A are active esters, it is preferable that this operation be carried out.

Here, the operation of reacting the active ester group with the low-molecular weight compound having the amino group is described especially as "blocking" in some cases. The surface of the medium after the carboxyl group or the active ester is reacted with the low-molecular weight compound is desirably hydrophilic. This is because a hydrophilic surface generally has an effect of suppressing nonspecific adsorption of biologically-relevant substances. Therefore, it is preferable that as the low-molecular weight compound containing the amino group, the low-molecular weight compound further having a hydrophilic group other than the amino group be used. Unlimited examples of such a low-molecular weight compound include ethanolamine, trishydroxymethylaminomethane and diglycolamine (IUPAC name: 2-(2-aminoethoxy)ethanol). Such a low-molecular weight compound is dissolved in 10 to 1,000 mmol/L in a buffer solution such as PBS, and the solution is brought into contact with the medium to which the temperature-responsive Protein A is fixed. For example, the reaction temperature may be set in the range of 4 to 37°C; and the reaction time, in the range of 2 min to 16 hours.

In the case where the temperature-responsive Protein A is fixed on the bead-shape medium, the temperature-responsive Protein A-fixed medium as the stationary phase may be packed and used in a commercially available empty column or an empty column fabricated from a glass tube. A commercially available empty column with a jacket (trade name: XK Column, GE Healthcare Japan Co., Ltd.), since being capable of being optionally controlled in the temperature of the column itself by controlling the temperature of a jacket circulating water, can suitably be used. In the case where the medium is of the film shape, the medium may be fixed and used on a commercially available film holder, or may be processed and used into an optional module shape, according to its film shape.

The temperature-responsive Protein A-fixed medium is preserved at a low temperature of about 2 to 10°C in a neutral solution in the range of a pH of 4 to 8 as a preservation medium. The preservation medium is preferably a 20% ethanol in consideration of antibacterial activity.

In the case where the antibody is purified from a mixture containing the antibody using the stationary phase having the temperature-responsive Protein A described hitherto, the mixture containing the antibody contains supernatants of culture solutions of hybridomas producing the antibody, myeloma cells such as NSO, animal cells being transformed with a gene to code the antibody and being capable of developing and producing the antibody, yeasts, and the like. Preferably, when the purification is carried out by the method according to the present embodiment, these culture supernatants are previously clarified. The clarification may be carried out, for example, by filtration using a membrane filter of 0.2 $\mu$m.

The antibody purified in the present embodiment is not limited, but includes, for example, human antibodies, non-human animal antibodies of ungulates and the like such as mice, cattle, goats and sheep, chimera antibodies of human and non-human animals, and humanized antibodies of non-human animal antibodies, and is preferably human antibodies, and is more preferably human monoclonal antibodies. Further, the class and subclass of the antibody are not limited, and an antibody of any class and subclass can be purified in the present embodiment, but preferable is IgG, and among these, preferable are IgG1, IgG2 and IgG4. Further the antibody may be an antibody in which at least one amino acid of the amino acids of the heavy chain constant domain present in nature is lost, in an amino acid sequence of the heavy chain constant domain, an antibody in which at least one amino acid of the amino acids of the heavy chain constant domain present in nature is replaced by another amino acid, therein, or an antibody in which at least one amino acid is added to the heavy chain constant domain present in nature, therein. Further, the antibody may be covalently or coordinately bonded with other compounds.

The temperature-responsive Protein A has a property of binding an antibody at a low temperature and releasing the antibody at a temperature higher than the temperature when binding the antibody. It is preferable that a temperature at which the binding property of the temperature-responsive Protein A to the antibody changes be previously ascertained, and the antibody be adsorbed on/desorbed from the stationary phase by changing the temperature so as to interpose the ascertained temperature. The temperature range where the antibody and the temperature-responsive Protein A are bound is a temperature range where the binding amount of the antibody becomes 50% or larger with respect to a maximum binding amount of the antibody capable of being bound to a predetermined amount of the stationary phase having the antibody; and the temperature range where the antibody is released from the temperature-responsive Protein A is a temperature range where the binding amount of the antibody becomes smaller than 50% with respect to the maximum binding amount of the antibody capable of being bound to the predetermined amount of the stationary phase having the antibody.

The temperature range where the antibody and the temperature-responsive Protein A are bound, and the temperature range where the antibody is released from the temperature-responsive Protein A are determined, for example by the following procedure.

1. Under each temperature condition of lower than 5°C, 10°C, and subsequently temperatures set at 10°C intervals up to right under a temperature at which the antibody is denatured, the antibody is bound to the stationary phase having the temperature-responsive Protein A.

2. The antibody is eluted from the stationary phase by raising the temperature up to a temperature right under a temperature at which the antibody is denatured, and qualitatively determined.

3. A plot of the eluting amounts of the antibody vs. the temperatures when the antibody was adsorbed on the stationary phase is made. Then, a temperature (hereinafter, referred to as "50% binding temperature") at an intersection point between a line of 50% of a maximum value of the binding amount (eluting amount) of the antibody and the plot is taken as a demarcation, and a temperature range imparting a binding amount of 50% or larger of the antibody is taken as the temperature range where the antibody and the temperature-responsive Protein A are bound. By contrast, a temperature range imparting a binding amount of smaller than 50% of the antibody is taken as the temperature range where the antibody is released from the temperature-responsive Protein A.

The temperature range where the antibody and the temperature-responsive Protein A are bound is, for example, a temperature range of 0°C or higher and lower than 20°C, preferably 1°C or higher and lower than 15°C, and more preferably 2°C or higher and lower than 13°C. The temperature range where the antibody is released from the temperature-responsive Protein A is, for example, a temperature range of 25°C or higher and lower than 50°C, and preferably 30°C or higher and lower than 45°C. Alternatively, the temperature range where the antibody is released from the temperature-responsive Protein A is preferably a temperature range of 15°C or higher and lower than 30°C, and more preferably 20°C or higher and lower than 25°C, from the viewpoint of enhancing the removability of impurities and suppressing the detachment of the Protein A. Here, although it is considered that the recovery rate of the antibody in the case of using the temperature range of 15°C or higher and 30°C or lower is lower than that in the case of using the temperature range of 25°C or higher and lower than 50°C, according to the present embodiment, the recovery rate of the antibody can be maintained while the removability of impurities is enhanced and the detachment of the Protein A is suppressed, by reducing the hydrogen ion exponent of the buffer solution used in the elution step of the antibody.

Here, as described above, in the method for purifying the antibody using the temperature-responsive Protein A according to the present embodiment, the hydrogen ion exponent of the buffer solution used in the elution step is set lower than that of the buffer solution used in the washing step. The present inventors have found that under this condition, there can be used a buffer solution at a temperature of, for example 0°C or higher and lower than 20°C as the temperature range where the antibody and the temperature-responsive Protein A are bound, and a buffer solution at a temperature of, for example, 15°C or higher and lower than 50°C (no reduction of the recovery rate even using the buffer solution at a temperature of 15°C or higher and 30°C or lower) as the temperature range where the antibody is released from the temperature-responsive Protein A. Although the temperature range where the antibody and the temperature-responsive Protein A are bound and the temperature range where the antibody is released from the temperature-responsive Protein A are partially superposed, this is permitted if there is a difference in the hydrogen ion exponent between the washing step and the elution step as described above. The temperature of the buffer solution in the elution step may be higher than that of the buffer solution in the washing step.

The culture temperature of Chinese hamster ovary (CHO) cells used in production of the monoclonal antibody is usually 37°C, and if the temperature of eluting the antibody from the temperature-responsive Protein A is set at lower than 37°C, the reduction of the activity of the antibody can be avoided. At this time, the temperature range suitable for releasing the antibody from the temperature-responsive Protein A is a temperature range of 10°C or higher and lower than 37°C, and preferably 15°C or higher and lower than 30°C.

A buffer solution containing the antibody eluted from the stationary phase having the temperature-responsive Protein A may further be purified by cation-exchange chromatography. In the cation-exchange chromatography, preferably, a stationary phase is used which contains a temperature-responsive cation-exchange resin to adsorb the antibody at a high temperature, and release the antibody at a low temperature.

As the stationary phase containing the temperature-responsive cation-exchange resin, a temperature-responsive cation-exchanger can be used in which a copolymer containing N-isopropylacrylamide and the like is fixed on the surface of a medium. The copolymer has at least cation-exchange groups. The temperature-responsive cation-exchanger according to the present embodiment is formed, for example, by polymerizing a monomer mixture composed of a monomer having a cation-exchange group and/or a cation-exchange group-introduced precursor monomer, and an N-isopropylacrylamide monomer, on the surface of the medium by a surface graft polymerization method. EP1281436 discloses a temperature-responsive cation exchange medium.

The shape of the medium used in the temperature-responsive cation-exchanger according to the present embodiment is not especially limited, and is, for example, of a bead shape, a flat plate shape or a tube shape. In the case of the bead shape, beads having various particle diameters are commercially available and are not especially limited, but the particle diameter is, for example, 1 to 300 μm, preferably 10 to 200 μm, and more preferably 20 to 150 μm. When the particle diameter is 1 μm or smaller, since the compaction of beads is liable to occur in a column, the treatment at a high speed is likely to become difficult. By contrast, when the particle diameter is 300 μm or larger, the gaps between beads become large, and the leakage of the solution is likely to occur when the antibody is adsorbed.

The medium has, for example, a plurality of pores. The pore diameter is not especially limited, but is, for example, 5 to 1,000 nm, preferably 10 to 700 nm, and more preferably 20 to 500 nm. When the pore diameter is 5 nm or smaller, the molecular weight of antibodies separable is likely to become low. By contrast, when the pore diameter is 1,000 nm or larger, the surface area of the medium becomes small, and the binding capacity of the antibody is likely to become low.

The material of the medium is not especially limited, but in the case of the bead shape, usable are glass, silica, polystyrene resins, methacryl resins, crosslinked agalose, crosslinked dextran, crosslinked polyvinyl alcohols, crosslinked cellulose, and the like.

In the present embodiment, the temperature-responsive polymer having cation-exchange groups is fixed on the medium. The fixing method includes an "atom transfer radical method" in which an atom transfer radical polymerization initiator is fixed on the surface of the medium, and a temperature-responsive polymer is grown and reacted from the initiator in the presence of a catalyst, and a "radiation-induced graft polymerization method" in which radicals are produced by irradiation with a radiation on the medium, and a temperature-responsive polymer is grown and reacted with the produced radicals as a starting point, but is not especially limited. Additionally, the fixing method includes the "atom transfer radical polymerization method" which is a surface living radical polymerization method. The "atom transfer radical polymerization method", since being capable of fixing the polymer in a high density on the surface of the medium, can suitably be used.

In the case where the temperature-responsive polymer is fixed by the "atom transfer radical polymerization method", an initiator used there is not especially limited, but includes, in the case where the medium has hydroxyl groups as in the present embodiment, for example, 1-trichlorosilyl-2-(m,p-chloromethylphenyl)ethane, 2-(4-chlorosulfonylphenyl)ethyltrimethoxysilane, (3-(2-bromoisobutylyl)propyl)dimethylethoxysilane and 2-bromoisobutyric acid bromide. In the present embodiment, the polymer chain is made to grow from the initiator. The catalyst at this time is not especially limited, but includes CuICl and CuIBr as copper halides (CuIX). Further, a ligand complex to the copper halide is not especially limited, but includes tris(2-dimethylamino)ethyl)amine (Me6TREN), N,N,N'',N''-pentamethyldiethylenetriamine (PMDETA), 1,1,4,7,10,10-hexamethyltriethylenetetramine (HMTETA), 1,4,8,11-tetramethyl-1,4,8,11-azacyclotetradecane (Me4Cyclam) and bipyridine.

In the case where the temperature-responsive polymer is fixed by the "radiation-induced graft polymerization method", any means can be employed in order to produce the radicals on the medium, but in order to produce radicals uniformly on the entire medium, the irradiation with an ionizing radiation is preferable. The kinds of the ionizing radiation utilizable are γ rays, electron beams, β rays, neutron beams and the like, but electron beams and γ rays are preferable for practicing in industrial scales. The ionizing radiation can be obtained from a radioisotope such as cobalt-60, strontium-90 or cesium-137, or an X-ray radiographic apparatus, an electron accelerator, an ultraviolet irradiation apparatus or the like.

The exposure dose of the ionizing radiation is, for example, 1kGy or more and 1,000 kGy or less, preferably 2 kGy or more and 500 kGy or less, and more preferably 5 kGy or more and 200 kGy or less. With the exposure dose being less than 1 kGy, radicals are likely to be hardly uniformly produced. With the exposure dose exceeding 1,000 kGy, the physical strength of a medium is likely to be caused to decrease.

The graft polymerization method using irradiation with the ionizing radiation is usually broadly divided into a previous irradiation method in which the radicals are produced on the medium, and thereafter, the radicals are brought into contact with the reactive compound, and a simultaneous irradiation method in which the radicals are produced on the medium in the state that the film is being brought into contact with the reactive compound. In the present embodiment, any method may be applicable, but the previous irradiation method, which provides little oligomer, is preferable.

The solvent used in the polymerization in the present embodiment is not especially limited as long as being capable of dissolving homogeneously the reactive compound. Examples of such solvents include alcohols such as ethanol, isopropanol and t-butyl alcohol, ethers such as diethyl ether and tetrahydrofuran, ketones such as acetone and 2-butanone, water, and mixtures thereof.

In the present embodiment, the polymer to be covered on the surface of the medium has a temperature-responsive monomer such as N-isopropylacrylamide. Poly(N-isopropylacrylamide) is known to have a lower limit critical temperature at 32°C. The medium having the polymer introduced to its surface largely changes the surface property of hydrophilicity/hydrophobicity at the critical temperature. Hence, in the case of using the medium grafted or covered with poly(N-isopropylacrylamide) as the stationary phase of chromatography, a retaining force to a sample is resultantly developed depending on the temperature. As a result, the retaining behavior can be controlled by the temperature without changing the composition of an eluent.

In order to make the lower limit critical temperature to be 32°C or higher, the requirement can be achieved by the preparation by copolymerization of N-isopropylacrylamide with hydrophilic comonomers such as acrylamide, methacrylic acid, acrylic acid, dimethylacrylamide and vinylpyrrolidone which are more hydrophilic monomers than isopropylacrylamide. Then, if the lower limit critical temperature is intended to be 32°C or lower, the requirement can be achieved by the preparation by copolymerization with hydrophobic comonomers such as styrene, alkyl methacrylates and alkyl acrylates which are hydrophobic monomers.

A first method for imparting the strong cation-exchange groups such as sulfonic acid groups to the polymer to be covered on the surface of the medium includes a method in which when the temperature-responsive polymer chain to be covered on the surface of the medium is synthesized, a copolymerization is carried out with the monomer having the strong cation-exchange group being contained in the system. The monomer having the sulfonic acid group includes (meth)acrylamide alkylsulfonic acids being a constituting unit of the polymers having sulfonic acid.

A second method for imparting the strong cation-exchange groups to the polymer to be covered on the surface of the medium includes a method in which after the copolymerization is carried out with the monomer, having a "strong cation-exchange group-introduced precursor", being contained in the system, the precursor is converted to the sulfonic acid group. Here, the "strong cation-exchange group-introduced precursor" may include a "precursor of a strong cation-exchange group". Here, the "precursor of a strong cation-exchange group" is, for example, a strong cation-exchange group with a blocking group attached thereto. The monomer having the precursor of the sulfonic acid group includes phenylvinyl sulfonate and the like, but the present embodiment is not limited thereto.

A third method for imparting the strong cation-exchange groups to the polymer to be covered on the surface of the medium includes a method in which after the copolymerization is carried out with the monomer, having a functional group capable of imparting the strong cation-exchange group, being contained in the system, the functional group capable of imparting the strong cation-exchange group is converted to the sulfonic acid group. The monomer having a functional group capable of imparting the strong cation-exchange group includes glycidyl methacrylate. In the case of polymerizing the monomer having the strong cation-exchange group by a surface living radical polymerization method, although a sufficient polymerization speed cannot often be attained, with use of a strong cation-exchange group-introduced precursor monomer such as glycidyl methacrylate a sufficient polymerization speed can be attained.

In the present embodiment, the monomer composition in which the ratio of the monomer having the strong cation-exchange group and/or the strong cation-exchange group-introduced precursor monomer is 0.01 to 5% by mol with respect to N-isopropylacrylamide is polymerized by the surface graft polymerization method. The ratio is preferably 0.1 to 4% by mol, more preferably 0.2 to 3% by mol, still more preferably 0.3 to 2% by mol, and most preferably 0.5 to 1.5% by mol. If the ratio exceeds 5% by mol, the amount of a strong cation-exchange group to N-isopropylacrylamide in a copolymer becomes excessive. Hence, although the amount of an antibody adsorbed to a temperature-responsive cation-exchanger increases, it is likely to become difficult for the adsorbed antibody to be eluted by a temperature change. By contrast, if the ratio is lower than 0.01% by mol, since the amount of a strong cation-exchange group introduced is small, the amount of the antibody adsorbed is likely to become small.

In the present embodiment, the polymer covered on the surface of the medium undergoes hydration and dehydration by temperature changes, and the temperature range is 0°C or higher and lower than 80°C, preferably 5°C or higher and lower than 50°C, and more preferably 10°C or higher and lower than 45°C. If the temperature range exceeds 80°C, since a moving phase is water, the evaporation and the like are caused and the workability is likely to become poor. By contrast, if being lower than 0°C, the moving phase is likely to be frozen.

The column packed with the temperature-responsive cation-exchanger obtained in the present embodiment is utilized as a liquid chromatography system by attached to a usual liquid chromatography apparatus. At this time, a method of loading temperatures to the column packed with the temperature-responsive cation-exchanger is not especially limited, but the method includes installing the column packed with the temperature-responsive cation-exchanger with an aluminum block, a water bath, an air layer, a jacket or the like, whose temperature is made at a predetermined temperature.

In the case where the antibody is purified using the stationary phase having the above-mentioned temperature-responsive Protein A, for example, a catch-and-release method is used in which the target antibody is once adsorbed to the stationary phase containing the temperature-responsive cation-exchange resin, and thereafter, the adsorbed antibody is released by changing the property of the surface of the stationary phase by changing the temperature. At this time, the amount of the antibody adsorbed to the stationary phase may exceed an amount capable of being adsorbed to the stationary phase, or may not exceed the amount. In either case, the purifying method is one in which the antibody is once adsorbed to the stationary phase, and thereafter, the adsorbed antibody is released by changing the property of the surface of the stationary phase by changing the temperature.

The temperature range where the antibody is adsorbed on the temperature-responsive cation-exchange resin is a high-temperature range of, for example, 25°C or higher and lower than 50°C, and preferably 30°C or higher and lower than 45°C. The temperature range where the antibody is released from the temperature-responsive cation-exchange resin is a low-temperature range of, for example, 0°C or higher and lower than 20°C, preferably 1°C or higher and lower than 15°C, and more preferably 2°C or higher and lower than 13°C.

Other separating methods are not especially limited, but include a method in which a temperature at which the surface property of hydrophilicity/hydrophobicity of the stationary phase is previously ascertained, and the separation of impurities is carried out by changing the temperature so as to interpose the ascertained temperature. In this case, since the surface property of hydrophilicity/hydrophobicity of the stationary phase is largely changed only by the temperature change, a large difference in the time (retention time) at which a signal emerges is expected to be caused depending on solutes. In the present embodiment, the separation carried out so as to interpose the temperature at which the surface property of hydrophilicity/hydrophobicity of the stationary phase largely changes is one of most effective embodiments.

Hereinafter, the method for purifying the antibody according to the present embodiment will be described in each step.

1) A binding step of binding the antibody to the temperature-responsive Protein A of the stationary phase.

In the method for purifying the antibody according to the present embodiment, a mixture solution containing the antibody is cooled down to a temperature at which the antibody is adsorbed to the temperature-responsive Protein A, and thereafter fed to the affinity chromatography column having the stationary phase having the temperature-responsive Protein A. In this case, a temperature at which the temperature-responsive Protein A and the antibody are bound is previously ascertained, and the temperature of the mixture solution containing the antibody is regulated at the ascertained temperature.

There are cases where the mixture solution containing the antibody may contain impurities such as protease, and cases where the mixture solution is preserved at a low temperature. In the case where the preserving temperature of the mixture containing the antibody is in the temperature range where the temperature-responsive Protein A and the antibody are bound, the antibody may be adsorbed as it is to the temperature-responsive Protein A. It is also possible that a heat exchanger is arranged right on the upstream of the temperature-responsive Protein A column, and the temperature of the mixture solution containing the antibody is continuously regulated while the mixture solution containing the antibody is being loaded on the temperature-responsive Protein A column.

Alternatively, the temperature of the mixture solution containing the antibody can also be regulated by immersing the chromatography column in a constant-temperature water bath whose temperature is regulated at a predetermined temperature. The temperature of the mixture solution containing the antibody may be regulated, in addition to arrangement of a heat exchanger right on the upstream of a chromatography column, by immersing the chromatography column in a constant-temperature water bath whose temperature is regulated at a predetermined temperature.

2) A washing step of washing the stationary phase having the temperature-responsive Protein A

The stationary phase is washed using a buffer solution having a low temperature at which the antibody and the temperature-responsive Protein A are bound, and having a first salt concentration and a first pH. The buffer solution usable is a phosphate buffer solution, a trishydrochloric acid buffer solution, or the like. The temperature at which the antibody and the temperature-responsive Protein A are bound is, for example, 0°C or higher and lower than 20°C, preferably 0°C or higher and 15°C or lower, more preferably 1°C or higher and lower than 15°C, and still more preferably 2°C or higher and lower than 13°C. The first salt concentration is, for example, 150 to 1,000 mmol/L, preferably 250 to 800 mmol/L, and more preferably 350 to 600 mmol/L. The first pH is, for example, 7.5 to 9.0, preferably 7.6 to 9.0, more preferably 7.6 to 8.8, still more preferably 7.7 to 8.6, and further still more preferably 8.0 to 8.6. By setting the salt concentration and the pH of the buffer solution in the washing step in theses ranges, foreign substances such as host cell-originated proteins (HCP), deoxyribonucleic acids (DNA) and the like remaining in the column are suitably removed, and the purity of the antibody to be recovered later is enabled to be enhanced. Here, although it is preferable that both the salt concentration and the pH of the buffer solution be set in these ranges, the salt concentration or the pH alone may be set in these ranges.

3) An elution step of eluting the antibody captured by the stationary phase having the temperature-responsive Protein A

The antibody captured by the stationary phase is eluted by passing through the column a buffer solution having a temperature at which the antibody is released from the temperature-responsive Protein A, and having a second salt concentration and (or) a second pH. The temperature at which the antibody is released from the temperature-responsive Protein A is, for example, 25°C or higher and lower than 50°C, and preferably 30°C or higher and lower than 45°C. However, by setting the temperature of the buffer solution at as low a temperature as possible, the removability of impurities is enhanced and the detachment of the temperature-responsive Protein A from the medium is enabled to be suppressed. In order to enhance such an effect to the maximum, the temperature range where the antibody is released from the temperature-responsive Protein A is set, preferably at 15°C or higher and 30°C or lower, and more preferably 20°C or higher and 25°C or lower. The second salt concentration is lower than the first salt concentration, and is, for example, 0 to 1,000 mmol/L, preferably 0 to 300 mmol/L, more preferably 0 to 100 mmol/L, and most preferably 0 mmol/L. The second pH is lower than the first pH, and is, for example, 5.0 to 8.0, preferably 5.0 to 7.0, and more preferably 5.0 to 6.5. By setting the salt concentration and the pH of the buffer solution in the elution step in these ranges, the detachment of the temperature-responsive Protein A from the medium is enabled to be suppressed. Hence, mingling of the temperature-responsive Protein A into an eluent of the antibody is enabled to be suppressed. Here, although it is preferable that both the salt concentration and the pH of the buffer solution be set in these ranges, the salt concentration or the pH alone may be set in these ranges.

As described above, if the temperature where the antibody is eluted from the temperature-responsive Protein A is made to be lower than 37°C, the reduction of the activity of the antibody can be avoided. The present inventors have found that when the temperature range where the antibody is released from the temperature-responsive Protein A is set at 10°C or higher and lower than 37°C, preferably 10°C or higher and 30°C or lower, more preferably 15°C or higher and 30°C or lower, and still more preferably 20°C or higher and 25°C or lower, the recovery rate can be raised by setting the second pH condition on the low side. The second pH condition is, for example, a pH of 3.0 to 8.0, preferably a pH of 3.5 to 7.0, more preferably a pH of 3.9 to 6.5, and still more preferably a pH of 4.0 or higher and 6.0 or lower.

4) An adsorption step of adsorbing the antibody to the stationary phase containing the cation-exchange resin

The buffer solution containing the antibody eluted from the stationary phase having the temperature-responsive Protein A is fed to the cation-exchange chromatography column having the stationary phase containing the cation-exchange resin while the temperature, the salt concentration and the pH were held at the same. Then, the antibody is adsorbed to the stationary phase containing the cation-exchange resin. Here, as described above, if the salt concentration of the buffer solution when the antibody captured by the stationary phase having the temperature-responsive Protein A is eluted is made lower than the salt concentration of the buffer solution when the antibody is bound to the stationary phase having the temperature-responsive Protein A, the buffer solution containing the antibody eluted from the stationary phase having the temperature-responsive Protein A is enabled to be fed as it is without the buffer solution being desalted.

The buffer solution containing the antibody eluted from the stationary phase having the temperature-responsive Protein A can be temporarily stored in a tank or the like, but the temperature of the buffer solution is preferably kept to such an degree that the temperature does not depart from the temperature range where the antibody is bound to the stationary phase containing the cation-exchange resin.

5) An elution step of eluting the antibody captured by the stationary phase containing the cation-exchange resin

The antibody captured by the stationary phase is eluted by passing through the column a buffer solution having a low temperature at which the antibody is released from the cation-exchange resin. The low temperature at which the antibody is released from the cation-exchange resin is, for example, 0°C or higher and lower than 20°C, preferably 1°C or higher and lower than 15°C, and more preferably 2°C or higher and lower than 13°C. When the antibody is eluted from the stationary phase containing the cation-exchange resin, for example, a heat exchanger is arranged right on the upstream of the temperature-responsive cation-exchange column, and the buffer solution at a predetermined temperature may be passed continuously. The antibody can also be eluted by immersing the temperature-responsive cation-exchange column in a constant-temperature water bath regulated at a predetermined temperature. The antibody can also be eluted not only using the heat exchanger arranged right on the upstream of the temperature-responsive cation-exchange column, but also by further immersing the temperature-responsive cation-exchange column in the constant-temperature water bath regulated at the predetermined temperature.

Here, before 1) the binding step of binding the antibody to the temperature-responsive Protein A of the stationary phase, an equilibration step may be provided in which the buffer solution of a low salt concentration and a high hydrogen ion exponent is brought into contact with the stationary phase having the temperature-responsive Protein A. The salt concentration of the buffer solution in the equilibration step is, for example, 0 to 1,000 mmol/L, preferably 0 to 250 mmol/L, and more preferably 0 to 100 mmol/L. The pH of the buffer solution in the equilibration step is, for example, 5.0 to 9.0, preferably 6.0 to 9.0, and more preferably 7.0 to 9.0. By setting the salt concentration and the pH of the buffer solution in the equilibration step in these ranges, the binding capacity of the antibody of the stationary phase having the temperature-responsive Protein A in the binding step thereafter is enabled to be increased.

However, in the case where the affinity column having the stationary phase having the temperature-responsive Protein A is repeatedly used, a buffer solution of the same salt concentration and pH as those of the buffer solution in 5) the elution step of eluting the antibody captured by the stationary phase containing the cation-exchange resin may be brought into contact with the stationary phase having the temperature-responsive Protein A in the equilibration step.

[Examples]

Hereinafter, the present embodiment will be described in more detail by way of examples.

(Preparation of a medium of temperature-responsive Protein A)

Carboxyl groups are introduced to crosslinked polyvinyl alcohol beads, and thereafter, the carboxyl groups are NHS-activated. Further by bringing the NHS-activated crosslinked polyvinyl alcohol beads into contact with temperature-responsive Protein A, the temperature-responsive Protein A is fixed to the crosslinked polyvinyl alcohol beads. The detail is as follows.

1) Introduction of carboxyl groups

A reaction solution was prepared in which 3.0 g of succinic anhydride and 3.6 g of 4-dimethylaminopyridine were dissolved in 450 mL of toluene. Then, 8.5 g of crosslinked polyvinyl alcohol beads (average particle diameter: 100 μm) prepared by the method described in Example 1 in Japanese Patent Laid-Open No. 59-17354 were brought into contact with the reaction solution at 50°C, and stirred for 2 hours. Thereby, carboxyl groups were introduced to the crosslinked polyvinyl alcohol beads. Thereafter, the crosslinked polyvinyl alcohol beads were washed with dehydrated isopropyl alcohol.

2) NHS activation

3 mL of the carboxyl group-introduced beads was charged in an NHS-activation reaction solution (NHS: 0.09 g, dehydrated isopropyl alcohol: 60 mL, diisopropylcarbodiimide: 0.12 mL), and allowed to react at 40°C for 30 min to thereby NHS-activate carboxyl groups on the bead surface. After the reaction, the beads were washed with ice-cooled dehydrated isopropyl alcohol, and further washed with 1 mM ice-cooled hydrochloric acid.

3) Coupling of temperature-responsive Protein A

A temperature-responsive Protein A was prepared by reference to Example 11 in Patent Literature (WO2008/143199). 150 mg of the temperature-responsive Protein A was dissolved in 3 mL of a coupling buffer solution (a phosphate buffer solution of 0.2 mol/L, NaCl of 0.5 mol/L, pH: 8.3) to thereby prepare a temperature-responsive Protein A solution. Then, the NHS-activated beads were charged in the temperature-responsive Protein A solution, and allowed to react at 25°C for 4 hours under shaking. After the elapse of a predetermined time, the beads were washed with the coupling buffer solution to wash and recover the temperature-responsive Protein A not having been coupling-reacted with the NHS-activated groups on the medium.

4) Blocking

The beads coupled with the temperature-responsive Protein A were immersed in 10 mL of a blocking reaction solution (ethanolamine of 0.5 mol/L, NaCl of 0.5 mol/L, pH: 8.0), and allowed to stand at room temperature for 30 min to thereby block the residual NHS with ethanolamine. After the reaction, the beads were washed with pure water, and preserved at 4°C in the state that a 20% ethanol is filled in a column.

Example 1

(Purification of an antibody by the temperature-responsive Protein A medium)

The temperature-responsive Protein A medium was packed in an empty column (GE Healthcare Japan Co., Ltd., Tricorn 5/20 column). The packing method was used by reference to the handling explanatory leaflet of the supplier. Then, the column was loaded on a chromatography system (GE Healthcare Japan Co., Ltd., AKTA FPLC).

A culture supernatant containing impurities was prepared by clarifying a culture solution of Chinese hamster oval (CHO) cells cultured at 37°C and adding an amount corresponding to 1 mg/mL of a polyclonal antibody (Benesis Co., Ltd., donated blood venoglobulin IH) to the clarified culture solution. A culture solution of CHO cells (cell density: about 8.9 x $10^6$/mL, living cell ratio: 66%) cultured in a serum-free culture medium (Irvine Scientific Co., IS CHO-CD medium) was used as the CHO cell culture solution, and filtered using a membrane filter (Asahi Kasei Medical Co., Ltd., trade name: BioOptimal(R): MF-SL) to thereby obtain the culture supernatant. The filtration was carried out by reference to the handling explanatory leaflet of the supplier.

Then, the culture supernatant containing the antibody was injected in the column under the following condition to thereby adsorb the antibody to the medium. Further the column was washed under the following condition to thereby elute the antibody from the column. Here, the pH of a washing buffer solution was made lower than the pH of an elution buffer solution.

1-1) Adsorption step

Antibody concentration: 1 mg/mL

Equilibrating buffer solution: a 20 mM phosphate buffer solution (pH: 7.5)
• Equilibration: 10-bead volume (used an adsorbing buffer solution)
• Antibody loading amount: 11 mL
• Flow rate: 0.4 mL/min
• Bead volume: 0.55 mL
• Adsorbing temperature: 2°C

1-2) Washing step

• Washing buffer solution: a 20 mM phosphate buffer solution (pH: 7.5)
• Flow rate: 0.4 mL/min
• Washing temperature: 2°C

1-3) Elution step

• Elution buffer solution: 20 mM phosphate buffer solution (pH: 7.0)

- Flow rate: 0.4 mL/min
- Permeated solution volume: 20 mL
- Eluting temperature: 40°C

(Measurement of the concentration of an antibody)

The concentration of an antibody contained in an eluting solution was measured using ultraviolet absorption at 280 nm (UV absorption), and calculated using the following expression (1).

$$\text{Antibody concentration (mg/mL)} = \text{absorbance} / 1.38 \quad \ldots \quad (1)$$

(Measurement of the concentration of a host cell protein (HCP))

The HCP concentration contained in an eluting solution was measured using a commercially available HCP measuring kit (Cygnus Co., CHO Host Cell Proteins 3rd. Generation ELISA Kit, catalog No. F550). The measurement was carried out by reference to the explanatory leaflet of the supplier. When the amount of HCP contained per 1 mg of an antibody before purification is taken to be $C_1$, and the amount of HCP contained in 1 mg of the antibody after the purification is taken to be $C_2$, the HCP removability by the purification can be represented by a logarithmic removal coefficient (LRV). Here, the logarithmic removal coefficient was calculated using the following expression (2).

$$\text{Logarithmic removal coefficient (LRV)} = \text{Log}_{10}(C_1/C_2) \quad \ldots \quad (2)$$

(Measurement of the concentration of a DNA)

The DNA concentration contained in an eluting solution was measured using a commercially available DNA assay kit (Invitrogen Corp., Qubit(R) dsDNA HS Assay Kit) and the measuring device (Invitrogen Corp., Qubit(R) Fluorometer). The measurement was carried out by reference to the explanatory leaflet of the supplier. When the amount of DNA contained per 1 mg of an antibody before purification is taken to be $C_3$, and the amount of DNA contained in 1 mg of the antibody after the purification is taken to be $C_4$, the DNA removability by the purification can be represented by a logarithmic removal coefficient (LRV). Here, the logarithmic removal coefficient was calculated using the following expression (3).

$$\text{Logarithmic removal coefficient (LRV)} = \text{Log}_{10}(C_3/C_4) \quad \ldots \quad (3)$$

(Method for measuring the Protein A content)

The content of Protein A contained in an eluting solution was measured using a commercially available Protein A assay kit (Cygnus Co., Protein A ELISA Kit, catalog No. F400). The measurement was carried out by reference to the handling explanatory leaflet (Immunoenzymetric Assay for the Measurement of Protein A Catalog #F400) attached to the assay kit, but steps 1 to 4 in the protocol described in page 4 of the explanatory leaflet were carried out in a cold chamber (10°C), and the other steps were carried out at room temperature.

The results of the above purification test are collectively described in Table 1. As a result, the HCP removability and the DNA removability were sufficiently high, and also the content of the Protein A contained in the eluted fraction was sufficiently low.

Example 2

The purification of the antibody was carried out by the same method as in Example 1, except for using a 20 mM phosphate buffer solution (pH: 8.0) as the washing buffer solution. Also in Example 2, the pH of the elution buffer solution was lower than that of the washing buffer solution. As a result, as shown in Table 1, the HCP removability and the DNA removability were sufficiently high, and also the content of the Protein A contained in the eluted fraction was sufficiently low.

Example 3

The purification of the antibody was carried out by the same method as in Example 1, except for using a 20 mM phosphate buffer solution (pH: 9.0) as the washing buffer solution. Also in Example 3, the pH of the elution buffer solution was lower than that of the washing buffer solution. As a result, as shown in Table 1, the HCP removability and the DNA removability were sufficiently high, and also the content of the Protein A contained in the eluted fraction was sufficiently low.

Example 4

The purification of the antibody was carried out by the same method as in Example 1, except for using a 20 mM phosphate buffer solution + a 150 mM NaCl (pH: 7.0) as the washing buffer solution. Therefore, in Example 4, the pH of the washing buffer solution and that of the elution buffer solution were the same, but the salt concentration of the elution buffer solution was lower than that of the washing buffer solution. As a result, as shown in Table 1, the HCP removability and the DNA removability were sufficiently high, and also the content of the Protein A contained in the eluted fraction was sufficiently low.

Example 5

The purification of the antibody was carried out by the same method as in Example 1, except for using a 20 mM phosphate buffer solution + a 300 mM NaCl (pH: 7.0) as the washing buffer solution. Therefore, in Example 5, the pH of the washing buffer solution and that of the elution buffer solution were the same, but the salt concentration of the elution buffer solution was lower than that of the washing buffer solution. As a result, as shown in Table 1, the HCP removability and the DNA removability were sufficiently high, and also the content of the Protein A contained in the eluted fraction was sufficiently low.

Example 6

The purification of the antibody was carried out by the same method as in Example 1, except for using a 20 mM phosphate buffer solution + a 450 mM NaCl (pH: 7.0) as the washing buffer solution. Therefore, in Example 6, the pH of the washing buffer solution and that of the elution buffer solution were the same, but the salt concentration of the elution buffer solution was lower than that of the washing buffer solution. As a result, as shown in Table 1, the HCP removability and the DNA removability were sufficiently high, and also the content of the Protein A contained in the eluted fraction was sufficiently low.

Example 7

The purification of the antibody was carried out by the same method as in Example 1, except for using a 20 mM phosphate buffer solution + a 150 mM NaCl (pH: 8.0) as the washing buffer solution. Therefore, in Example 7, the pH of the elution buffer solution was lower than that of the washing buffer solution. The salt concentration of the elution buffer solution was lower than that of the washing buffer solution. As a result, as shown in Table 1, the HCP removability and the DNA removability were sufficiently high, and also the content of the Protein A contained in the eluted fraction was sufficiently low.

Example 8

The purification of the antibody was carried out by the same method as in Example 1, except for using a 20 mM phosphate buffer solution + a 150 mM NaCl (pH: 9.0) as the washing buffer solution. Therefore, in Example 8, the pH of the elution buffer solution was lower than that of the washing buffer solution. The salt concentration of the elution buffer solution was lower than that of the washing buffer solution. As a result, as shown in Table 1, the HCP removability and the DNA removability were sufficiently high, and also the content of the Protein A contained in the eluted fraction was sufficiently low.

Example 9

The purification of the antibody was carried out by the same method as in Example 1, except for using a 20 mM phosphate buffer solution + a 150 mM NaCl (pH: 7.0) as the washing buffer solution, and a 20 mM phosphate buffer solution + a 150 mM NaCl (pH: 6.0) as the elution buffer solution. Therefore, in Example 9, the pH of the elution buffer solution was lower than that of the washing buffer solution, but the salt concentration of the washing buffer solution and that of the elution buffer solution were the same. As a result, as shown in Table 1, the HCP removability and the DNA removability were sufficiently high, and also the content of the Protein A contained in the eluted fraction was sufficiently low.

Example 10

The purification of the antibody was carried out by the same method as in Example 1, except for using a 20 mM phosphate buffer solution + a 150 mM NaCl (pH: 7.0) as the washing buffer solution, and a 20 mM phosphate buffer solution + a 150 mM NaCl (pH: 5.0) as the elution buffer solution. Therefore, in Example 10, the pH of the elution buffer solution was lower than that of the washing buffer solution, but the salt concentration of the washing buffer solution and that of the elution buffer solution were the same. As a result, as shown in Table 1, the HCP removability and the DNA removability were sufficiently high, and also the content of the Protein A contained in the eluted fraction was sufficiently low.

Example 11

The purification of the antibody was carried out by the same method as in Example 1, except for using a 20 mM phosphate buffer solution + a 150 mM NaCl (pH: 7.0) as the washing buffer solution, and a 20 mM phosphate buffer solution (pH: 8.0) as the elution buffer solution. Therefore, in Example 11, the pH of the elution buffer solution was higher than that of the washing buffer solution, but the salt concentration of the elution buffer solution was lower than that of the washing buffer solution. As a result, as shown in Table 1, the HCP removability and the DNA removability were sufficiently high, and also the content of the Protein A contained in the eluted fraction was sufficiently low.

Example 12

The purification of the antibody was carried out by the same method as in Example 1, except for using a 20 mM phosphate buffer solution + a 150 mM NaCl (pH: 7.0) as the washing buffer solution, and a 20 mM phosphate buffer solution (pH: 7.0) as the elution buffer solution. Therefore, in Example 12, the pH of the washing buffer solution and that of the elution buffer solution were the same, but the salt concentration of the elution buffer solution was lower than that of the washing buffer solution. As a result, as shown in Table 1, the HCP removability and the DNA removability were sufficiently high, and also the content of the Protein A contained in the eluted fraction was sufficiently low.

Example 13

The purification of the antibody was carried out by the same method as in Example 1, except for using a 20 mM phosphate buffer solution + a 150 mM NaCl (pH: 7.0) as the washing buffer solution, and a 20 mM phosphate buffer solution (pH: 6.0) as the elution buffer solution. Therefore, in Example 13, the pH of the elution buffer solution was lower than that of the washing buffer solution. The salt concentration of the elution buffer solution was lower than that of the washing buffer solution. As a result, as shown in Table 1, the HCP removability and the DNA removability were sufficiently high, and also the content of the Protein A contained in the eluted fraction was sufficiently low.

Example 14

The purification of the antibody was carried out by the same method as in Example 1, except for using a 20 mM phosphate buffer solution + a 150 mM NaCl (pH: 7.0) as the washing buffer solution, and a 20 mM phosphate buffer solution (pH: 5.0) as the elution buffer solution. Therefore, in Example 14, the pH of the elution buffer solution was lower than that of the washing buffer solution. As a result, as shown in Table 1, the HCP removability and the DNA removability were sufficiently high, and also the content of the Protein A contained in the eluted fraction was sufficiently low.

Example 15

A temperature-responsive cation-exchange resin having sulfonic acid groups was synthesized by an atom transfer radical polymerization method. Then, the antibody purified in Example 13 was purified by the temperature-responsive cation-exchange resin.

1) Fixation of an initiator

1 g of the crosslinked polyvinyl alcohol beads (particle diameter: 100 $\mu$m) was moistened with pure water, and charged in a 300-mL glass-made conical flask. 200 mL of tetrahydrofuran (containing no stabilizer, made by Kanto Chemical Co., Inc.), 1.23 mL of 2-bromoisobutyric acid bromide (made by Tokyo Chemical Industry Co., Ltd.), and 1.40 mL of triethylamine (made by Wako Pure Chemical Industries, Ltd.) were added to the conical flask, and shaken at room temperature for 16 hours. After the reaction, the resultant was filtered and three times washed with 200 mL of ethanol, and preserved in dehydrated isopropanol. Thereby, the 2-bromoisobutyric acid bromide as an initiator for the atom transfer radical polymerization (ATRP) was introduced on the surface of the crosslinked polyvinyl alcohol beads.

2) Surface graft polymerization

A monomer composition was prepared in which glycidyl methacrylate (GMA, made by Tokyo Chemical Industry Co., Ltd.) as a precursor monomer of a sulfonic acid group was contained in a proportion of 1% by mol with respect to N-isopropylacrylamide. Specifically, 18.40 g of N-isopropylacrylamide (IPAAm, made by Wako Pure Chemical Industries, Ltd.), 0.231 g of GMA, 1.217 g of butyl methacrylate (BMA, made by Tokyo Chemical Industry Co., Ltd.), 0.085 g of copper I chloride (CuCl, made by Wako Pure Chemical Industries, Ltd.), and 0.012 g of copper II chloride (CuCl$_2$, made by Wako Pure Chemical Industry Co., Ltd.) were dissolved in 42.8 mL of a 90-vol% isopropanol (IPA) solution, and bubbled with nitrogen for 30 min. Thereafter, 0.221 g of tris(2-dimethylaminoethyl)amine (Me$_6$TREN)(made by Alfa Aesar Co.) was added to the solution in a nitrogen atmosphere, and stirred for 5 min to thereby form a catalyst of CuCl/CuCl$_2$/Me$_6$TREN. The reaction solution was reacted with the initiator-introduced crosslinked polyvinyl alcohol beads in a nitrogen atmosphere and ATRP was carried out at room temperature for 16 hours. After the reaction, the resultant was washed with, ethanol, a 50-mmol/L EDTA aqueous solution, and pure water in this order to thereby wash the monomer, the polymer and the copper catalyst.

3) Introduction of a sulfonic acid group

The beads having graft chains introduced by the atom transfer radical polymerization method were charged in 200 g of a mixed aqueous solution of sodium sulfite and IPA (sodium sulfite/IPA/pure water = 10/15/75 in % by weight), and allowed to react at 80°C for 24 hours to thereby convert epoxy groups in the graft chains to sulfonic acid groups. After the reaction, the beads were washed with pure water. Thereafter, beads were charged in a 0.5 mol/L sulfuric acid and allowed to react at 80°C for 2 hours to thereby convert the remaining epoxy groups in the graft chains to diol groups. After the reaction, the beads were washed with pure water to thereby make a temperature-responsive adsorbent relevant to Example 1.

4) Measurement of a copolymerization ratio

A monomer composition was used in which glycidyl methacrylate (GMA, made by Tokyo Chemical Industry Co., Ltd.) as a precursor monomer of a sulfonic acid group was contained in a proportion of 1% by mol with respect to N-isopropylacrylamide, and a copolymer was polymerized without using a base material. Specifically, the reaction solution described in the above 2) was reacted with ethyl 2-bromoisobutyrate in a nitrogen atmosphere and ATPR was carried out at room temperature for 16 hours. After the reaction, the reaction solution was put in a dialysis membrane (Spectra/por Dialysis Membrane, MWCO1000, made by Spectrum Laboratories Inc.), and immersed in ethanol, a 50-mmol/L EDTA aqueous solution and pure water in this order to thereby remove the monomer and the copper catalyst. Then, a copolymer obtained by freeze-drying the reaction solution was charged in 200 g of a mixed aqueous solution of sodium sulfite and IPA (sodium sulfite/IPA/pure water = 10/15/75 in % by weight), and allowed to react at 80°C for 24 hours to thereby convert epoxy groups in the graft chains to sulfonic acid groups. After the reaction, the reaction solution was put in a dialysis membrane, immersed in pure water to remove sodium sulfite and IPA, and freeze-dried the reaction solution to thereby obtain a copolymer.

30 mg of the above copolymer was dissolved in 670 mg of heavy water, and the copolymer was measured by 1H-NMR using a nuclear magnetic resonance apparatus (Bruker Corp., Avenve-600). Thereafter, a copolymerization ratio (composition) of the monomer unit having the strong cation-exchange group with respect to N-isopropylacrylamide was calculated from a signal-integrated value originated from N-isopropylacrylamide units and a signal-integrated value originated from sulfonic aid groups. As a result, the copolymerization ratio (composition) of the monomer unit having the strong cation-exchange group with respect to N-isopropylacrylamide was 0.72% by mol.

5) Measurements of the adsorbing and eluting amounts of the antibody protein

The beads were packed in an empty column (Tricorn 5/20 column, made by GE Healthcare Japan Co., Ltd.), and adsorption and elution tests of the antibody protein (donated blood venoglobulin IH, made by Benesis Co., Ltd.,) with the temperature change were carried out using a chromatography system (AKTA FPLC, made by GE Healthcare Japan Co., Ltd.). The temperature-change operation of the column packed with the beads was carried out by stopping for a while a pump of the chromatography system, immersing the column in a constant-temperature water bath, thereafter allowed to stand for 10 min or longer, and thereafter restarting the pump of the chromatography system. The adsorption and the elution of the antibody protein were carried out under the following conditions.

(Adsorption step)

· Antibody protein concentration: 2.5 mg/mL
· Adsorbing buffer: a 20 mM phosphate buffer solution (pH: 6.0)
· Antibody protein solution loading amount: 20 mL
· Flow rate: 0.4 mL/min
· Column volume: 0.54 mL
· Adsorbing temperature: 40°C

(Washing step)

· Washing buffer: a 20 mM phosphate buffer solution (pH: 6.0)
· Flow rate: 0.4 mL/min
· Washing temperature: 40°C

(Temperature-elution step)

· Elution buffer: a 20 mM phosphate buffer solution (pH: 6.0)
· Flow rate: 0.4 mL/min
· Flow volume: 20 mL
· Eluting temperature: 2°C

(Salt-elution step)

· Elution buffer: a 20 mM phosphate buffer solution + a 1 M NaCl (pH: 6.0)
· Flow rate: 0.4 mL/min
· Flow volume: 20 mL
· Eluting temperature: 2°C

After the temperature-elution, the antibody protein not completely having been eluted by the temperature was eluted using a 20 mM phosphate buffer solution + a 1M NaCl (pH: 6.0). The UV absorptions (280 nm) of fractions of each step were measured; the antibody protein concentrations were calculated; and the temperature-eluting amount of the antibody protein was calculated.

(The result)

The temperature-eluting amount of the antibody protein was 30.7 mg/mL, which indicated that the antibody protein can be eluted by the temperature change. The antibody protein remaining on the beads after the temperature-elution was eluted with a salt buffer, and the salt-eluting amount was as small as 1.4 mg/mL. Form the above result, it was indicated that the antibody protein could be purified industrially using the temperature-responsive cation-exchange resin with no need of exchanging buffer solutions, after the purification using the temperature-responsive Protein A.

Example 16

The purification of the antibody was carried out by the same method as in Example 1, except for using a 20 mM phosphate buffer solution + a 300 mM NaCl (pH: 8.0) as the washing buffer solution, and a 50 mM citrate buffer solution + a 300 mM NaCl (pH: 3.0) as the elution buffer solution, and carrying out the purification at an eluting temperature of 25°C. Therefore, in Example 16, the pH of the elution buffer solution was lower than that of the washing buffer solution. As a result, as shown in Table 1, the HCP removability and the DNA removability were sufficiently high, and also the content of the Protein A contained in the eluted fraction was sufficiently low. The eluting temperature of the antibody was 25°C, and the elution was carried out in the temperature range of avoiding the inactivation by high temperatures. Further the recovery rate of the antibody was calculated by the following expression (4), and was as sufficiently high as 100%.

(Measurement of the concentration of the antibody)

The antibody concentration contained in the eluent was measured using ultraviolet absorption (UV absorption) at 280 nm, and calculated by the following expression (4).

```
Recovery rate (%) = (an antibody concentration in
the fraction in the elution step (mg/mL) × (an amount of
the fraction in the elution step (mL)) × 100 / ((an
antibody concentration in the fed solution in the
adsorption step (mg/mL) × an amount of the washadsorption
step (mL)) - (an antibody concentration of the fraction
in the adsorption step (mg/mL)) × an amount of fraction
in the adsorption step (mL)) - (an antibody concentration
in the fraction in the washing step (mg/mL)) × an amount
of the fraction in the washing step (mL))) ... (4)
```

Example 17

The purification of the antibody was carried out by the same method as in Example 1, except for using a 20 mM phosphate buffer solution + a 300 mM NaCl (pH: 8.0) as the washing buffer solution, and a 50 mM citrate buffer solution + a 300 mM NaCl (pH: 4.0) as the elution buffer solution, and carrying out the purification at an eluting temperature of 25°C. Therefore, in Example 17, the pH of the elution buffer solution was lower than that of the washing buffer solution. As a result, as shown in Table 1, the HCP removability and the DNA removability were sufficiently high, and also the content of the Protein A contained in the eluted fraction was sufficiently low. The eluting temperature of the antibody was 25°C, and the elution was carried out in the temperature range of avoiding the inactivation by high temperatures. The recovery rate of the antibody was 99%.

Example 18

The purification of the antibody was carried out by the same method as in Example 1, except for using a 20 mM phosphate buffer solution + a 300 mM NaCl (pH: 8.0) as the washing buffer solution, and a 50 mM citrate buffer solution + a 300 mM NaCl (pH: 5.0) as the elution buffer solution, and carrying out the purification at an eluting temperature of 25°C. Therefore, in Example 18, the pH of the elution buffer solution was lower than that of the washing buffer solution. As a result, as shown in Table 1, the HCP removability and the DNA removability were sufficiently high, and also the content of the Protein A contained in the eluted fraction was sufficiently low. The eluting temperature of the antibody was 25°C, and the elution was carried out in the temperature range of avoiding the inactivation by high temperatures. The recovery rate of the antibody was 100%.

[Example 19]

The purification of the antibody was carried out by the same method as in Example 1, except for using a 20 mM phosphate buffer solution + a 150 mM NaCl (pH: 7.0) as the washing buffer solution, and a 20 mM phosphate buffer solution + a 300 mM NaCl (pH: 6.0) as the elution buffer solution. As a result, as shown in Table 1, the HCP removability and the DNA removability were sufficiently high.

[Example 20]

The purification of the antibody was carried out by the same method as in Example 1, except for using a 20 mM phosphate buffer solution + a 300 mM NaCl (pH: 8.0) as the washing buffer solution, and a 50 mM citrate buffer solution (pH: 4.0) as the elution buffer solution, and carrying out the purification at an eluting temperature of 25°C. Therefore, in Example 20, the pH of the elution buffer solution was lower than that of the washing buffer solution, and the salt concentration of the elution buffer solution was lower than that of the washing buffer solution, and the elution buffer solution contained no salt. As a result, as shown in Table 1, the HCP removability and the DNA removability were sufficiently high, and also the content of the Protein A contained in the eluted fraction was sufficiently low. The eluting temperature of the antibody was 25°C, and the elution was carried out in the temperature range of avoiding the inactivation by high temperatures. The recovery rate of the antibody was 99%.

[Comparative Example 1]

The purification of the antibody was carried out by the same method as in Example 1, except for using a 20 mM phosphate buffer solution (pH: 6.0) as the washing buffer solution. As a result, as shown in Table 1, since the pH of the washing buffer solution was higher than that of the elution buffer solution, the HCP removability and the DNA removability were low.

[Comparative Example 2]

The purification of the antibody was carried out by the same method as in Example 1, except for using a 20 mM phosphate buffer solution (pH: 7.0) as the washing buffer solution. As a result, as shown in Table 1, since the salt concentrations and the pHs of the washing buffer solution and the elution buffer solution were the same, the HCP removability and the DNA removability were low.

[Comparative Example 3]

The purification of the antibody was carried out by the same method as in Example 1, except for using a 20 mM phosphate buffer solution + a 100 mM NaCl (pH: 7.4) as the washing buffer solution and the elution buffer solution. As a result, as shown in Table 1, although the HCP removability and the DNA removability were sufficiently high, since the salt concentrations and the pHs of the washing buffer solution and the elution buffer solution were the same, the content of the Protein A contained in the elution fraction was high.

[Comparative Example 4]

The purification of the antibody was carried out by the same method as in Example 1, except for using a 20 mM phosphate buffer solution + a 150 mM NaCl (pH: 7.0) as the washing buffer solution, and a 20 mM phosphate buffer solution + a 150 mM NaCl (pH: 8.0) as the elution buffer solution. As a result, as shown in Table 1, although the HCP removability and the DNA removability were sufficiently high, since the salt concentrations of the washing buffer solution and the elution buffer solution were the same, and the pH of the elution buffer solution was higher than that of the washing buffer solution, the content of the Protein A contained in the elution fraction was high.

[Comparative Example 5]

The purification of the antibody was carried out by the same method as in Example 1, except for using a 20 mM phosphate buffer solution + a 150 mM NaCl (pH: 7.0) as the washing buffer solution and the elution buffer solution.

As a result, as shown in Table 1, although the HCP removability and the DNA removability were sufficiently high, since the salt concentrations and the pHs of the washing buffer solution and the elution buffer solution were the same, the content of the Protein A contained in the elution fraction was high.

[Example 21]

The purification of the antibody was carried out by the same method as in Example 1, except for carrying out the purification at an eluting temperature of 25°C. The recovery rate of the antibody was as low as 24%.

[Table 1]

| | | Washing Buffer Solution | Elution Buffer Solution | HCP Removability (LRV) | DNA Removability (LRV) | Protein A Content (ng/mL) |
|---|---|---|---|---|---|---|
| Example 1 | | 20 mM phosphate buffer solution (pH7.5) | 20 mM phosphate buffer solution (pH7.0) | 1.55 | 1.69 | less than 10 |
| Example 2 | | 20 mM phosphate buffer solution (pH8.0) | 20 mM phosphate buffer solution (pH7.0) | 1.76 | 1.75 | less than 10 |
| Example 3 | | 20 mM phosphate buffer solution (pH9.0) | 20 mM phosphate buffer solution (pH7.0) | 1.92 | 1.88 | less than 10 |
| Example 4 | | 20 mM phosphate buffer solution + 150 mM NaCl (pH7.0) | 20 mM phosphate buffer solution (pH7.0) | 1.97 | 1.81 | less than 10 |
| Example 5 | | 20 mM phosphate buffer solution + 300 mM NaCl (pH7.0) | 20 mM phosphate buffer solution (pH7.0) | 2.22 | 2.35 | less than 10 |
| Example 6 | | 20 mM phosphate buffer solution + 450 mM NaCl (pH7.0) | 20 mM phosphate buffer solution (pH7.0) | 2.29 | 2.55 | less than 10 |
| Example 7 | | 20 mM phosphate buffer solution + 150 mM NaCl (pH8.0) | 20 mM phosphate buffer solution (pH7.0) | 2.21 | 2.02 | less than 10 |
| Example 8 | | 20 mM phosphate buffer solution + 150 mM NaCl (pH9.0) | 20 mM phosphate buffer solution (pH7.0) | 2.47 | 2.21 | less than 10 |
| Example 9 | | 20 mM phosphate buffer solution + 150 mM NaCl (pH7.0) | 20 mM phosphate buffer solution + 150 mM NaCl (pH6.0) | 1.91 | 1.85 | less than 10 |
| Example 10 | | 20 mM phosphate buffer solution + 150 mM NaCl (pH7.0) | 20 mM phosphate buffer solution + 150 mM NaCl (pH5.0) | 1.93 | 1.83 | less than 10 |
| Example 11 | | 20 mM phosphate buffer solution + 150 mM NaCl (pH7.0) | 20 mM phosphate buffer solution (pH8.0) | 1.92 | 1.87 | less than 10 |
| Example 12 | | 20 mM phosphate buffer solution + 150 mM NaCl (pH7.0) | 20 mM phosphate buffer solution (pH7.0) | 1.99 | 1.88 | less than 10 |
| Example 13 | | 20 mM phosphate buffer solution + 150 mM NaCl (pH7.0) | 20 mM phosphate buffer solution (pH6.0) | 1.92 | 1.91 | less than 10 |

(continued)

| | Washing Buffer Solution | Elution Buffer Solution | HCP Removability (LRV) | DNA Removability (LRV) | Protein A Content (ng/mL) |
|---|---|---|---|---|---|
| Example 14 | 20 mM phosphate buffer solution + 150 mM NaCl (pH7.0) | 20 mM phosphate buffer solution (pH5.0) | 1.94 | 1.82 | less than 10 |
| Example 16 | 20 mM phosphate buffer solution + 300 mM NaCl (pH8.0) | 50 mM citrate buffer solution + 300 mM NaCl (pH3.0) | 2.33 | 2.09 | less than 10 |
| Example 17 | 20 mM phosphate buffer solution + 300 mM NaCl (pH8.0) | 50 mM citrate buffer solution + 300 mM NaCl (pH4.0) | 2.23 | 2.01 | less than 10 |
| Example 18 | 20 mM phosphate buffer solution + 300 mM NaCl (pH8.0) | 50 mM citrate buffer solution + 300 mM NaCl (pH5.0) | 2.21 | 1.99 | less than 10 |
| Example 19 | 20 mM phosphate buffer solution + 150 mM NaCl (pH7.0) | 20 mM phosphate buffer solution + 300 mM NaCl (pH6.0) | 1.98 | 1.79 | 233 |
| Example 20 | 20 mM phosphate buffer solution + 300 mM NaCl (pH8.0) | 50 mM citrate buffer solution (pH4.0) | 2.75 | 2.85 | less than 10 |
| Comp. Ex. 1 | 20 mM phosphate buffer solution (pH6.0) | 20 mM phosphate buffer solution (pH7.0) | 0.89 | 1.34 | - |
| Comp. Ex. 2 | 20 mM phosphate buffer solution (pH7.0) | 20 mM phosphate buffer solution (pH7.0) | 1.11 | 1.41 | - |
| Comp. Ex. 3 | 20 mM phosphate buffer solution + 100 mM NaCl (pH7.4) | 20 mM phosphate buffer solution + 100 mM NaCl (pH7.4) | 1.69 | 1.67 | 666 |
| Comp. Ex. 4 | 20 mM phosphate buffer solution + 150 mM NaCl (pH7.0) | 20 mM phosphate buffer solution + 150 mM NaCl (pH8.0) | 1.92 | 1.75 | 1902 |
| Comp. Ex. 5 | 20 mM phosphate buffer solution + 150 mM NaCl (pH7.0) | 20 mM phosphate buffer solution + 150 mM NaCl (pH7.0) | 1.90 | 1.77 | 623 |

**Claims**

1. A method for purifying an antibody using temperature-responsive Protein A, comprising:

a binding step of binding the antibody to a stationary phase having the temperature-responsive Protein A;
a washing step of washing the stationary phase using a buffer solution having a first salt concentration at a temperature at which the temperature-responsive Protein A and the antibody are bound; and
an elution step of eluting the antibody captured by the stationary phase using a buffer solution having a second salt concentration lower than the first salt concentration at a temperature at which the antibody is released from the temperature-responsive Protein A,
wherein different buffer solutions are used in the washing step and the elution step.

2. The method for purifying an antibody according to claim 1, wherein the buffer solution used in the elution step has a lower hydrogen ion exponent than the buffer solution used in the washing step.

3. A method for purifying an antibody using temperature-responsive Protein A, comprising:

   a binding step of binding the antibody to a stationary phase having the temperature-responsive Protein A,
   a washing step of washing the stationary phase using a buffer solution having a first hydrogen ion exponent at a temperature at which the temperature-responsive Protein A and the antibody are bound; and
   an elution step of eluting the antibody captured by the stationary phase using a buffer solution having a second hydrogen ion exponent lower than the first hydrogen ion exponent at a temperature at which the antibody is released from the temperature-responsive Protein A,
   wherein the different buffer solutions are used in the washing step and the elution step.

4. The method for purifying an antibody according to claim 3, wherein the buffer solution used in the elution step has a lower salt concentration than the buffer solution used in the washing step.

5. The method for purifying an antibody according to any one of claims 1 to 4, wherein the buffer solution used in the washing step has a salt concentration of 150 to 1,000 mmol/L, and the buffer solution used in the elution step has a salt concentration of 0 to 1,000 mmol/L.

6. The method for purifying an antibody according to any one of claims 1 to 5, wherein the buffer solution in the washing step has a hydrogen ion exponent of 7.5 to 9.0, and the buffer solution in the elution step has a hydrogen ion exponent of 3.0 to 8.0.

7. The method for purifying an antibody according to any one of claims 1 to 6, wherein the buffer solution in the washing step has a temperature of 0 to 20°C.

8. The method for purifying an antibody according to any one of claims 1 to 7, wherein the buffer solution in the elution step has a temperature of 15 to 50°C.

9. The method for purifying an antibody according to any one of claims 1 to 8, wherein the buffer solution in the elution step has a higher temperature than the buffer solution in the washing step.

10. The method for purifying an antibody according to any one of claims 1 to 9, further comprising an adsorption step of bringing the buffer solution containing the antibody and being eluted from the stationary phase having the temperature-responsive Protein A into contact with a stationary phase containing a cation-exchange resin to thereby adsorb the antibody to the stationary phase containing the cation-exchange resin.

11. The method for purifying an antibody according to claim 10, wherein the buffer solution in the adsorption step has the same salt concentration and the same hydrogen ion exponent as the buffer solution in the elution step.

12. The method for purifying an antibody according to claim 10 or 11, wherein the buffer solution in the adsorption step has the same temperature as the buffer solution in the elution step.

13. The method for purifying an antibody according to any one of claims 10 to 12, wherein the cation-exchange resin is a temperature-responsive cation-exchange resin.

14. The method for purifying an antibody according to any one of claims 1 to 13, further comprising, before the binding step, an equilibration step of bringing a buffer solution having the same salt concentration and the same hydrogen ion exponent as the buffer solution in the elution step into contact with the stationary phase having the temperature-responsive Protein A.

15. The method for purifying an antibody according to any one of claims 1 to 13, further comprising, before the binding step, an equilibration step of bringing a buffer solution having a low salt concentration and a high hydrogen ion exponent into contact with the stationary phase having the temperature-responsive Protein A; wherein the low salt concentration is from 0 to 1000 mmol/L and the high hydrogen ion exponent is from 5.0 to 9.0.

**Patentansprüche**

1. Verfahren zur Reinigung eines Antikörpers unter Verwendung von temperaturabhängigem Protein A, umfassend:

einen Bindungsschritt, bei dem der Antikörper an eine stationäre Phase gebunden wird, die das temperaturabhängige Protein A aufweist;

einen Waschschritt, bei dem die stationäre Phase unter Verwendung einer Pufferlösung, die eine erste Salzkonzentration aufweist, bei einer Temperatur, bei der das temperaturabhängige Protein A und der Antikörper gebunden sind, gewaschen wird; und

einen Elutionsschritt, bei dem der durch die stationäre Phase abgefangene Antikörper unter Verwendung einer Pufferlösung, die eine zweite Salzkonzentration aufweist, welche niedriger ist als die erste Salzkonzentration, bei einer Temperatur, bei der der Antikörper von dem temperaturabhängigen Protein A freigesetzt wird, eluiert wird;

wobei im Waschschritt und im Elutionsschritt unterschiedliche Pufferlösungen verwendet werden.

2. Verfahren zur Reinigung eines Antikörpers gemäß Anspruch 1, wobei die im Elutionsschritt verwendete Pufferlösung einen niedrigeren pH-Wert aufweist als die im Waschschritt verwendete Pufferlösung.

3. Verfahren zur Reinigung eines Antikörpers unter Verwendung von temperaturabhängigem Protein A, umfassend:

einen Bindungsschritt, bei dem der Antikörper an eine stationäre Phase gebunden wird, die das temperaturabhängige Protein A aufweist;

einen Waschschritt, bei dem die stationäre Phase unter Verwendung einer Pufferlösung, die einen ersten pH-Wert aufweist, bei einer Temperatur, bei der das temperaturabhängige Protein A und der Antikörper gebunden sind, gewaschen wird; und

einen Elutionsschritt, bei dem der durch die stationäre Phase abgefangene Antikörper unter Verwendung einer Pufferlösung, die einen zweiten pH-Wert aufweist, der niedriger ist als der erste pH-Wert, bei einer Temperatur, bei der der Antikörper von dem temperaturabhängigen Protein A freigesetzt wird, eluiert wird;

wobei im Waschschritt und im Elutionsschritt unterschiedliche Pufferlösungen verwendet werden.

4. Verfahren zur Reinigung eines Antikörpers gemäß Anspruch 3, wobei die im Elutionsschritt verwendete Pufferlösung eine niedrigere Salzkonzentration aufweist als die im Waschschritt verwendete Pufferlösung.

5. Verfahren zur Reinigung eines Antikörpers gemäß einem der Ansprüche 1 bis 4, wobei die im Waschschritt verwendete Pufferlösung eine Salzkonzentration von 150 bis 1000 mmol/l aufweist und die im Elutionsschritt verwendete Pufferlösung eine Salzkonzentration von 0 bis 1000 mmol/l aufweist.

6. Verfahren zur Reinigung eines Antikörpers gemäß einem der Ansprüche 1 bis 5, wobei die Pufferlösung im Waschschritt einen pH-Wert von 7,5 bis 9,0 aufweist und die Pufferlösung im Elutionsschritt einen pH-Wert von 3,0 bis 8,0 aufweist.

7. Verfahren zur Reinigung eines Antikörpers gemäß einem der Ansprüche 1 bis 6, wobei die Pufferlösung im Waschschritt eine Temperatur von 0 bis 20 °C aufweist.

8. Verfahren zur Reinigung eines Antikörpers gemäß einem der Ansprüche 1 bis 7, wobei die Pufferlösung im Elutionsschritt eine Temperatur von 15 bis 50 °C aufweist.

9. Verfahren zur Reinigung eines Antikörpers gemäß einem der Ansprüche 1 bis 8, wobei die Pufferlösung im Elutionsschritt eine höhere Temperatur aufweist als die Pufferlösung im Waschschritt.

10. Verfahren zur Reinigung eines Antikörpers gemäß einem der Ansprüche 1 bis 9, weiterhin umfassend einen Adsorptionsschritt, bei dem die Pufferlösung, die den Antikörper enthält und die von der stationären Phase, welche das temperaturabhängige Protein A aufweist, eluiert wird, mit einer stationären Phase, die ein Kationenaustauscherharz enthält, in Kontakt gebracht wird, wodurch der Antikörper an der stationären Phase, die das Kationenaustauscherharz enthält, adsorbiert wird.

11. Verfahren zur Reinigung eines Antikörpers gemäß Anspruch 10, wobei die Pufferlösung im Adsorptionsschritt dieselbe Salzkonzentration und denselben pH-Wert aufweist wie die Pufferlösung im Elutionsschritt.

12. Verfahren zur Reinigung eines Antikörpers gemäß Anspruch 10 oder 11, wobei die Pufferlösung im Adsorptionsschritt dieselbe Temperatur aufweist wie die Pufferlösung im Elutionsschritt.

**13.** Verfahren zur Reinigung eines Antikörpers gemäß einem der Ansprüche 10 bis 12, wobei das Kationenaustauscherharz ein temperaturabhängiges Kationenaustauscherharz ist.

**14.** Verfahren zur Reinigung eines Antikörpers gemäß einem der Ansprüche 1 bis 13, vor dem Bindungsschritt weiterhin umfassend einen Äquilibrierungsschritt, bei dem eine Pufferlösung, die dieselbe Salzkonzentration und denselben pH-Wert aufweist wie die Pufferlösung im Elutionsschritt, mit der stationären Phase, die das temperaturabhängige Protein A aufweist, in Kontakt gebracht wird.

**15.** Verfahren zur Reinigung eines Antikörpers gemäß einem der Ansprüche 1 bis 13, vor dem Bindungsschritt weiterhin umfassend einen Äquilibrierungsschritt, bei dem eine Pufferlösung, die eine niedrige Salzkonzentration und einen hohen pH-Wert aufweist, mit der stationären Phase, die das temperaturabhängige Protein A aufweist, in Kontakt gebracht wird, wobei die niedrige Salzkonzentration 0 bis 1000 mmol/l beträgt und der hohe pH-Wert 5,0 bis 9,0 beträgt.

**Revendications**

**1.** Procédé de purification d'un anticorps en utilisant une protéine A réactive à la température, comprenant les étapes suivantes :

une étape de liaison qui lie l'anticorps à une phase fixe comportant la protéine A réactive à la température ;
une étape de lavage qui lave la phase fixe en utilisant une solution tampon ayant une première concentration en sel à une température à laquelle la protéine A réactive à la température et l'anticorps sont liés ; et
une étape d'élution pour éluer l'anticorps capturé par la phase fixe en utilisant une solution tampon ayant une seconde concentration en sel inférieure à la première concentration en sel à une température à laquelle l'anticorps est libéré de la protéine A réactive à la température,
où des solutions tampon différentes sont utilisées dans l'étape de lavage et dans l'étape d'élution.

**2.** Procédé de purification d'un anticorps selon la revendication 1, dans lequel la solution tampon utilisée dans l'étape d'élution possède un exposant en ion hydrogène inférieur à celui de la solution tampon utilisée dans l'étape de lavage.

**3.** Procédé de purification d'un anticorps en utilisant une protéine A réactive à la température, comprenant les étapes suivantes :

une étape de liaison qui lie l'anticorps à une phase fixe comportant la protéine A réactive à la température ;
une étape de lavage qui lave la phase fixe en utilisant une solution tampon ayant un premier exposant en ion hydrogène à une température à laquelle la protéine A réactive à la température et l'anticorps sont liés ; et
une étape d'élution pour éluer l'anticorps capturé par la phase fixe en utilisant une solution tampon ayant un second exposant en ion hydrogène inférieur au premier exposant en ion hydrogène à une température à laquelle l'anticorps est libéré de la protéine A réactive à la température,
où les différentes solutions tampon sont utilisées dans l'étape de lavage et l'étape d'élution.

**4.** Procédé de purification d'un anticorps selon la revendication 3, dans lequel la solution tampon utilisée dans l'étape d'élution a une concentration en sel inférieure à celle de la solution tampon utilisée dans l'étape de lavage.

**5.** Procédé de purification d'un anticorps selon l'une quelconque des revendications 1 à 4, dans lequel la solution tampon utilisée dans l'étape de lavage a une concentration en sel de 150 à 1 000 mmol/l, et la solution tampon utilisée dans l'étape d'élution a une concentration en sel de 0 à 1 000 mmol/l.

**6.** Procédé de purification d'un anticorps selon l'une quelconque des revendications 1 à 5, dans lequel la solution tampon de l'étape de lavage a un exposant en ion hydrogène de 7,5 à 9,0, et la solution tampon de l'étape d'élution a un exposant en ion hydrogène de 3,0 à 8,0.

**7.** Procédé de purification d'un anticorps selon l'une quelconque des revendications 1 à 6, dans lequel la solution tampon de l'étape de lavage a une température de 0 à 20 °C.

**8.** Procédé de purification d'un anticorps selon l'une quelconque des revendications 1 à 7, dans lequel la solution tampon de l'étape d'élution a une température de 15 à 50 °C.

9. Procédé de purification d'un anticorps selon l'une quelconque des revendications 1 à 8, dans lequel la solution tampon de l'étape d'élution a une température supérieure à celle de la solution tampon de l'étape de lavage.

10. Procédé de purification d'un anticorps selon l'une quelconque des revendications 1 à 9, comprenant en outre une étape d'adsorption pour amener la solution tampon contenant l'anticorps et étant éluée depuis la phase fixe ayant la protéine A réactive à la température en contact avec une phase fixe contenant une résine échangeuse de cations pour alors adsorber l'anticorps sur la phase fixe contenant la résine échangeuse de cations.

11. Procédé de purification d'un anticorps selon la revendication 10, dans lequel la solution tampon de l'étape d'adsorption a la même concentration en sel et le même exposant en ion hydrogène que la solution tampon de l'étape d'élution.

12. Procédé de purification d'un anticorps selon la revendication 10 ou 11, dans lequel la solution tampon de l'étape d'adsorption a la même température que la solution tampon de l'étape d'élution.

13. Procédé de purification d'un anticorps selon l'une quelconque des revendications 10 à 12, dans lequel la résine échangeuse de cations est une résine échangeuse de cations réactive à la température.

14. Procédé de purification d'un anticorps selon l'une quelconque des revendications 1 à 13, comprenant en outre, avant l'étape de liaison, une étape d'équilibrage pour mettre une solution tampon ayant la même concentration en sel et le même exposant en ion hydrogène que la solution tampon de l'étape d'élution en contact avec la phase fixe ayant la protéine A réactive à la température.

15. Procédé de purification d'un anticorps selon l'une quelconque des revendications 1 à 13, comprenant en outre, avant l'étape de liaison, une étape d'équilibrage pour mettre une solution tampon ayant une faible concentration en sel et un fort exposant en ion hydrogène en contact avec la phase fixe ayant la protéine A réactive à la température, où la faible concentration en sel est de 0 à 1 000 mmol/l et l'exposant élevé en ion hydrogène est de 5,0 à 9,0.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009196998 A
- JP 2007526897 A
- WO 2008143199 A
- WO 2011017514 A
- WO 2012086837 A
- EP 1281436 A
- JP 59017354 A